# NEW EUROPEAN PATENT SPECIFICATION

(11) **EP 0 478 626 B2**
(45) Date of publication and mention of the opposition decision: **27.06.2007**
(45) Mention of the grant of the patent: 17.01.1996
(21) Application number: 90909351.0
(22) Date of filing: 21.06.1990
(51) Int. Cl.: G01N 21/76

(54) **DETECTING OR QUANTIFYING MULTIPLE ANALYTES USING LABELLING TECHNIQUES**
ERMITTLUNG UND QUANTIFIZIERUNG MEHRERER ANALYTEN UNTER VERWENDUNG VON MARKIERUNGSTECHNIKEN
DETECTION OU QUANTIFICATION DE PLUSIEURS ANALYTES AU MOYEN DE TECHNIQUES D'ETIQUETAGE

(30) Priority: 24.06.1989 GB 8914563
(43) Date of publication of application: 08.04.1992
(73) Proprietor: GEN-PROBE INCORPORATED, San Diego, California 92121 (US)
(72) Inventor: BATMANGHELICH, Shariar, Cardiff CF2 5HN (GB); WOODHEAD, James, Stuart, Raglan NP5 2LD (GB); WEEKS, Ian, Cardiff CF3 9DJ (GB)
(74) Representative: Jaenichen, Hans-Rainer
(86) International application number: PCT/GB1990/000957
(87) International publication number: WO 1991/000511

(56) References cited:
- EP-B- 0 304 202
- EP-B- 0 377 729
- WO-A-89/01157
- WO-A-90/00164
- WO-A-90/00168
- GB-A- 2 008 247
- US-A- 3 352 791
- US-A- 3 352 791
- US-A- 4 713 348
- US-A- 4 713 348
- US-A- 4 931 223
- US-A- 4 931 223
- TRENDS IN ANALYTICAL CHEMISTRY, volume 7, no. 2, 1988, Elsevier Science Publishers B.V., J. Stuart Woodhead et al. : "Chemiluminescence immunoassays", pages 55-58.
- PATENT ABSTRACTS OF JAPAN, volume 9, no. 90 (P-350)(1813), 19 April 1985 ; & JP-A-59217136
- 5th international Symposium on Bioluminescence and hemiluminescence, Florence-Bologna: 25-29 Sept.1988, Poster
- 5th int. Symp. on Bioluminescence and Chemiluminescence, Final Programme; 25-29 Sept. 1988
- Journal of Bioluminescence and Chemiluminescence, Vol. 5, 1-4(1990), Edwards et al.
- The Journal of Histochem. and Cytochem.,25, 1977; Loken et al
- Confirmatory Affidavit by Brooks Edwards dated 25 Nov. 1966
- Clin. Chem., 32(5), 887, 1986, Wians et al.
- Clin. Chem., 29 (6), 1051, 1983, Dean et al.
- Cloin. Chem., 28(7), 1469,1982, Blake et al
- Clin. Chem., 33(12), 2281, 1987, Hemmila et al
- Clin. Chem., 29(8), 1474, 1983, Weeks et al
- Proceedings of the 3rd Quantitative Luminescence Spectrometry in Biomedical Sciences, Gent, Belgium, May 22-26,1989
- Accounts of Chemical Research, 9(6), 201, 1976, Mc Capra
- J. of Bioluminescence, 4, 51, 1989, McCapra et al

## Description

This invention relates to methods and reagents for the assay, detection, quantification, location or analysis of each of a plurality of substances of interest ("analytes") in a sample in which each substance is linked ("labelled") with another molecule or molecules capable of taking part in a chemiluminescent reaction.

For the purposes of this specification, a chemiluminescent reaction is defined as one which involves a chemical reaction that results in the emission of electromagnetic radiation. This luminescence is to be distinguished clearly from fluorescence and phosphorescence. Here, luminescence, or more precisely, chemiluminescence also encompasses light emission from biological reactions (bioluminescent reactions).

A luminescent reaction is normally one between at least two molecules (S and L) with or without other reagents, cofactors, or a catalyst (D) or under the influence of a physical trigger. L is the substance which generates light, such as luminol. S is the substance which reacts with L to cause excitation, for example oxygen or hydrogen peroxide. D (if present) is a cofactor, and/or catalyst or trigger such as an enzyme, a luciferase, or potassium ferricyanide. The reaction between L and S results in the conversion of L to an excited molecule L* and the return of this excited molecule to a non-excited state results in the emission of a photon. The reaction between L and S and the decay of L* to the non-excited state may take place spontaneously or may require the presence of the cofactor or catalyst D, or a physical trigger such as temperature. An example of such a reaction is the oxidation by H₂O₂ of luminol. The catalyst and cofactors are often inorganic compounds as here, but may also be extracted from biological material such as the enzyme peroxidase which catalyses the luminescent reaction involving luminol.

These methods and reagents discussed above may be used in a wide variety of techniques such as immuno-assays, protein binding assays, nucleic acid hybridisation assays, cellular receptor binding assays and other analogous techniques which involve binding of the substance of interest with a specific binding partner or reagent. These types of linking are referred to herein as "binding or otherwise linking with".

The substances of interest may be peptides, proteins, polypeptides, nucleic acids and other substances of biological interest.

Binding assays have been used for many years in the quantitation of molecules of biological interest. Numerous examples, have been described in which the binding step is an immunological reaction, a protein binding reactiion, reaction with a cellular receptor or a complementary nucleic acid hybridisation reaction. Sensitive assays based on these reactions require the use of a label which can be attached or incorporated into one of the binding partners of such a reaction such that the degree of binding and hence the concentration or mass of another component of the reaction - the substance of interest - can be determined. Many variations of the basic binding reactions have been described and many different labels used, including radioisotopes, enzymes, fluorescent molecules and chemiluminescent molecules.

Various combinations of these have been used in sequence for the detection and quantitation of a wide variety of analytes ranging from small molecules such as hormones and drugs to large molecules such as nucleotides.

Generally speaking, these techniques have only been applied to the investigation of a single analyte in one test reaction, but there have been a limited number of examples where two analytes have been determined essentially using a single test procedure. The best known of these have been simultaneous immunoassays and/or protein binding assays for vitamin B12 and folic acid and also for thyroxine and thyrotrophin. In these cases the two different reactions are monitored independently using a different radioactive isotope for each. Here use is made of cobatt-57 and iodine-125 whose radioactive emissions are distinguishable using an appropriate gamma counter. Similar strategies have also been used for the simultaneous determination of lutrophin and follitrophin. Wians et al., Clin. Chemistry, Vol. 32, No. 5, 1986 relates to radioimmunoassay and involves Lutropin (LH) and follicle stimulating hormone (FSH) being simultaneously quantified, the assay kit exploiting the difference in scintillation energies produced by ⁵⁷Co and ¹²⁵I labelled tracers.

Radioactive reagents have three major disadvantages. Firstly, the method of labelling involves the use of highly radioactive and hence potentially hazardous reagents. Secondly, the shelf life of the radioactively labelled substance is often relatively short not only because by its very nature the radioactive isotope is continuously decaying, but also radioactively labelled proteins are often unstable. Thirdly, it is often difficult to label proteins sufficiently to provide a sensitively and rapidly detectable reagent The measurement of luminesence is both highly sensitive and very rapid, the time of measurement being of the order of seconds rather than the several minutes normally required for measurement of radioactivity. The attachment either covalently or non-cavalentty, to substances not normally capable of taking part in a luminesescent reaction of a substance which is capable of taking part in a luminescent reaction provides a reagent which can be rapidly measured in very small quantities. Weeks et al., Clin. Chemistry, Vol. 29, No. 8, 1983 reports on the synthesis of a (one) stable chemiluminescent acridinium ester derivative that is capable of covalent association with an antibody. Proteins so labelled can be detected at lower concentrations than can the corresponding ₁₂₅I derivatives. It is remarked that the universal application of chemiluminescent labels makes them a logical alternative to radioisotopes for immunoassay purposes.

Work has been described relating to the use of different fluorescent molecules in so-called "dual labelling" systems. However, fluorescent labelling systems are usually capable of only gross analysis of substances and are not generally suitable for sensitive analysis. Also, with fluroescent systems, the sample is illuminated by U.V. radiation to measure the fluoresence arid this may cause major problems due to photobleaching. Multiple analyte immunoassays based on the use of fluorophores have been described in which the different labels used have been chelates of different lanthanide metals emitting at different wavelengths. Limitations arise here because certain of the fluorophores used have low quantum yields and generally all assays based on these materials require complex instrumentation and chemistries in order to achieve the high levels of performance which are characteristic of many chemiluminescent systems (Ref.1).

Broadly stated, according to one aspect of this invention, there is provided a method for the assay, detection, quantification, location or analysis of each of a plurality of substances of interest contained in a sample, which comprises labelling each of said substances with one or more components capable of taking part in a respective distinguishable chemiluminescent reaction.

This invention provides a method for the assay, detection, quantification, location or analysis of a sample containing at least two substances of interest comprising the steps of
i) reacting said sample with a mixture comprising at least a first reagent and a second reagent that form at least a first complex and a second complex wherein said first complex comprises one of said substances or a respective associated substance and said first reagent, and wherein said second complex comprises another said substance and said second reagent, wherein at least said first reagent and second reagent each comprise
   a) a binding partner for binding or otherwise linking with one or more of said substances, and
   b) a different chemiluminescent molecule exhibiting distinguishable emission characteristics, wherein said chemiluminescent molecule is chemically or physically coupled to said binding partner;
ii) subsequently treating said sample comprising said first and second complexes to cause a first and second chemiluminescent reaction to occur, wherein said chemiluminescent reactions are simultaneously triggered, and
iii) observing, sensing, measuring and/or recording the emissions of each of said chemiluminescent reactions.
In another aspect, this invention provides a luminescent reagent which comprises a mixture of at least two reagents, wherein said reagents each comprise
a) a binding partner for binding or otherwise linking with respective substances of interest; and
b) a different chemiluminescent molecule exhibiting distinguishable emission characteristics, chemically or physically coupled to the binding partner.

We have found that different chemiluminescent labels can be produced which, by appropriate chemical manipulation, possess different characteristics in terms of the speed and wavelength of light emission and that these different labels can be used advantageously in analyte binding systems to permit the substantially simultaneous quantitation of two or more different analytes within a single test procedure.

It is known that certain changes in the structure of chemiluminescent molecules cause changes in the spectrum of the emitted light Recently it has been suggested that conventional enzyme immunoassays with light emitting end points could be used for multiple assays (Ref.2) though it is not taught how this may be achieved nor is it obvious how such assays would be performed. Neither are there any data to support this suggestion. No comparable suggestions have been made for situations in which luminescent molecules themselves are linked to substances of biological interest as opposed to using luminescent end-point enzyme immuno-assays. The advantages of using such direct labelling as opposed to enzymic modulation are well established and yield advantages in terms of simplicity, robustness and sensitivity. No teachings exist as to how the advantages of such systems may be exploited for multiple analyte assays nor is it obvious what instrumentation would be required etc. in order to perform such assays. More recently it has been shown that modification of the luciferase component of bioluminescent reactions by genetic manipulation (Ref.3) forms an additional means of modifying the wavelength of emission from such reactions as an alternative to modifying the chemical structure of the corresponding luciferin.

In a further aspect it is known that, generally, change in chemical reaction conditions or structural changes within the reactant molecules themselves may affect the rates of their various possible reactions. Similarly, more specifically, the kinetics of chemiluminescent reactions are often affected by the chemiluminescent molecule. It has been suggested for example (Ref. 4,5) that the rates of chemiluminescent reactions of acridinium salts are dependent on certain structural features. Likewise it has been reported that enzyme driven chemiluminescent reactions may be affected by the structure of the substates (Ref.6). That these kinetic effects may be utilised in the development of multiple assays based on such kinetic differences has not been taught, neither is it obvious from any related art how such assays may be configured and utilised.

A discussion of non-limiting embodiments of the invention now follow, together with a specific example of an assay procedure, reference being made to the accompanying drawings, in which:-
Figure 1 is a schematic graph showing emission intensity vs. time a typical test procedure according to the invention; and
Figure 2 is a schematic graph showing emission intensity vs. time in an example of procedure according to the invention for the immunochemiluminometric assay for human gonadotrophin and human alpha-fetoprotein.

### General Scheme

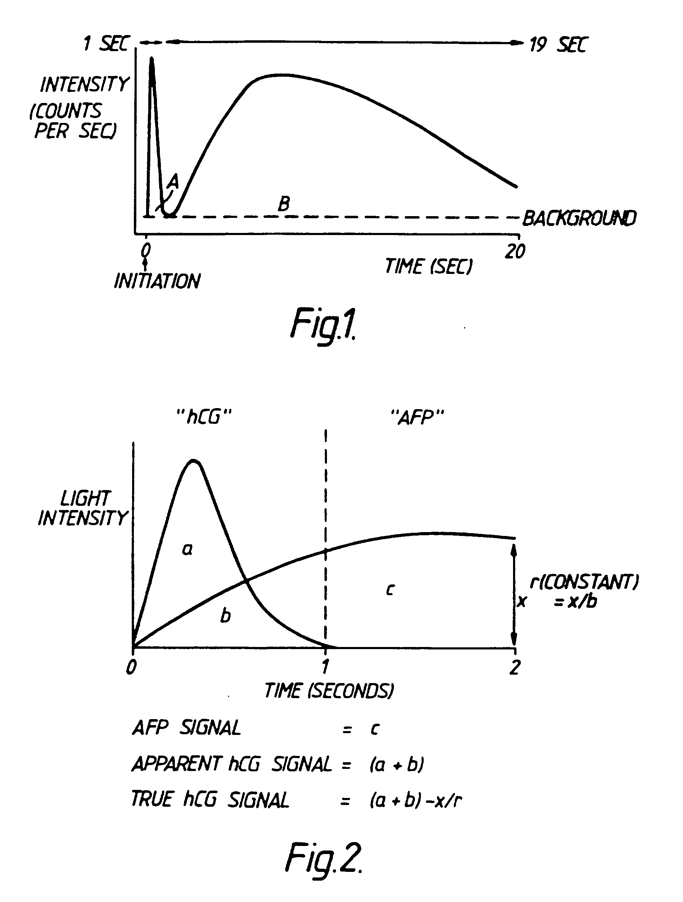

A and B are the analytes of interest and are each capable of binding more than one antibody molecule so that parallel two-site immunoassays can be set up. A mixture of antibodies capable of binding A and B is coated on to the walls of a test tube. The sample for analysis containing unknown amounts of A and B is added to the tube together with a mixture of soluble complementary antibodies capable of binding to other sites on A and B. The soluble antibodies specific for A and B are labelled with chemiluminescent molecules exhibiting distinguishable characteristics, e.g. fast and slow light emission. Following an appropriate incubation period, two-site immune complexes will be formed on the sides of the tube, the extent of immune complex formation depending on the amount of A and B present. Following removal of unbound substances by aspiration of the soluble contents of the tube, the chemiluminescence emission remaining is triggered and then measured in a luminometer. The total number of photons emitted is proportional to the total amount of A and B present. However, in this example, the chemiluminescence emission from labelled antibodies specific for A is rapid and complete within one second whereas the emission from labelled antibodies specific for B is much slower and reaches a peak after initiation before decaying over the next nineteen seconds (see Figure 1). Thus measurement of the photons emitted in two separate time windows of 1 and 19 seconds within an overall measuring time of 20 seconds permits independent quantitation of A and B upon calibration of the system.

### Assay Techniques and Analytes

The methods in accordance with the invention may quantify those species of biological interest which are identifiable using single analyte quantitation techniques. The following examples are given with guidance as to the type of binding reaction used. This list is given for example only and does not imply any limitations of the invention:-
1. Immunoassay:
   Drugs, vitamins, steroids, thyroid hormones, peptides, polypeptides, proteins, immunoglobulins, viruses, bacteria, protozoa.
2. Protein binding assays:
   Vitamins, cofactors, enzyme inhibitors.
3. Nucleic acid hybridisation assays:
   Oligonucleotides, polynucleotides, DNA, RNA, onocogenes, microorganisms.
4. Receptor binding assays:
   Progestogen receptors, estrogen receptors, thyrotrophin receptors, thyroid hormone receptors.

Certain pairs of groups of analytes are often measured to get a more complete picture of the biological system or to improve efficiency of testing of the biological system. In such cases the availability of simultaneous, multi-analyte measurement offered by the invention is uniquely advantageous. Examples of such groups of analytes are given below but do not imply limitations of the invention.
1. Hormones:
   Thyroxin/thyrotrophin, lutrophin/follitrophin, adrenocorticortrophin/cortisol.
2. Vitamins and cofactors:
   Vitamin B12/folic acid, 1,25-dihydroxycholicalciferol/25-hydroxycholecalciferol.
3. Nucleic acids (from viruses and micro-organisms):
   Neisseria Gonorrhoea/Chlamydia Trachomatis.
4. Tumour markers:
   Prostate Specific Antigen/Prostatic Acid Phosphatase, alphafetoprotein/carcinoembryonic antigen/chorionic gonadotrophin.

### Labels

The preferred way of associating chemiluminesence activity with the appropriate binding reaction is to chemically or physically couple a component such as a chemiluminescent molecule, capable of taking part in a chemiluminescent reaction, to one of the components of that binding reaction so as to produce a specific labelled reagent. The luminescent reagents according to the present invention will thus include two or more such labelled reagents each carrying a label having different characteristics in terms of kinetic and/or spectroscopic properties. Each of these labelled reagents will have a particular specificity for taking part in a given binding reaction, thus each given binding reaction can be monitored independently even though two or more such reactions are occurring simultaneously. Hence it is possible to quantify, independently and simultaneously, the analytes taking part in these parallel binding reactions.

Different members of a number of classes of chemiluminescent molecules are capable of exhibiting differences in kinetic and/or spectroscopic properties and can hence be used in the invention, including acridinium and related compounds (e.g. phenanthridinium compounds), phthalhydrazides and related compounds (e.g. naphthalhydrazides), oxalate esters and related compounds and also stabilised dioxetanes and dioxetanones. The variations of compounds within such groups are well-known to those moderately skilled in the art, likewise it is known that the quantum yield, kinetics and emission wavelengths of their chemiluminescent reactions are affected by their structure (see earlier and also Refs.7-11). Thus the structures of a plurality of compounds differing in kinetics or emission wavelength parameters, suitable for use in this invention are individually readily conceived by one skilled in the art. For example, from the existing literature, one skilled in the art would know to choose from those compounds with high quantum yields, and to choose those compounds which, relative to each other possessed substantial differences in their reaction rates or their emission wavelengths, in order to maximise the resolution between the detection of these compounds. Aryl acridinium esters may be used as labels with appropriate chemical modifications made to produce the desired kinetic and spectroscopic parameters. Some examples of such compounds are given below and do not imply any limitations of the invention.
1. Kinetic variation
a. 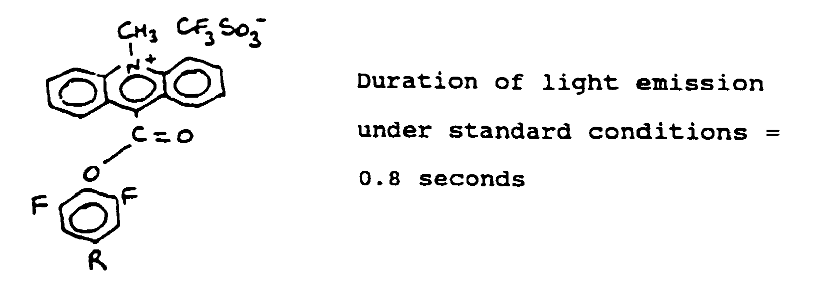
b. 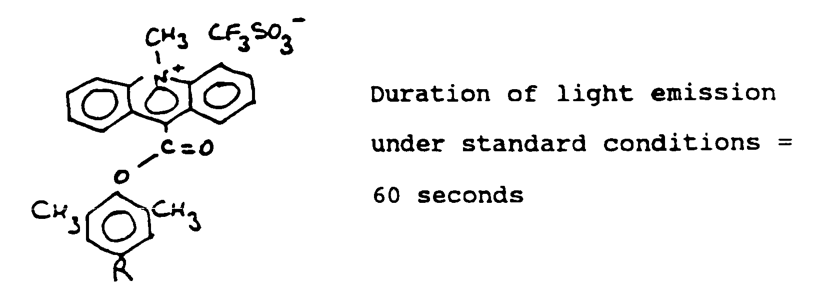
In the acridinium phenyl ester of 1a. the phenyl moiety is substituted with F groups which are electron withdrawing and thus modify the acridinium phenyl ester so that the emission of light occurs over a relatively short period. In the acridinium phenyl ester of 1b, the phenyl moiety is substituted with CH₃ groups which.are electron donating so that the emission of light occurs over a relatively long period. Other electron donating and withdrawing groups may be used.
c. 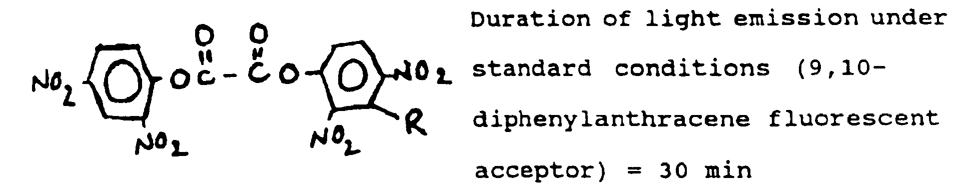
d. 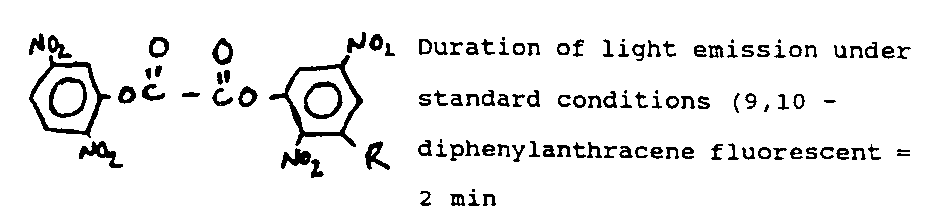 Table 1 gives further examples for the series

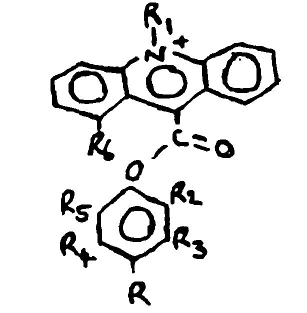

| R₁ | R₂ | R₃ | R₄ | R₅ | R₆ | t½ (decayphase half-life) |
|---|---|---|---|---|---|---|
| CH₃ | H | H | H | H | H | 0.6s |
| CH₃ | CH₃O | H | H | CH₃O | H | 7s |
| C₆H₅CH₂ | H | H | H | H | H | 0.5s |
| CH₃ | CH₃ | H | H | H | H | 11s |
| CH₃ | NO₂ | H | H | NO₂ | H | < 0.4s |
| CH₃ | H | CH₃ | CH₃ | H | H | 0.7s |
| CH₃ | CH₃ | H | H | CH₃ | CH₃ | 4s |
| CH₃ | Br | H | H | Br | H | < 0.4s |

2. Spectroscopic variation
a. 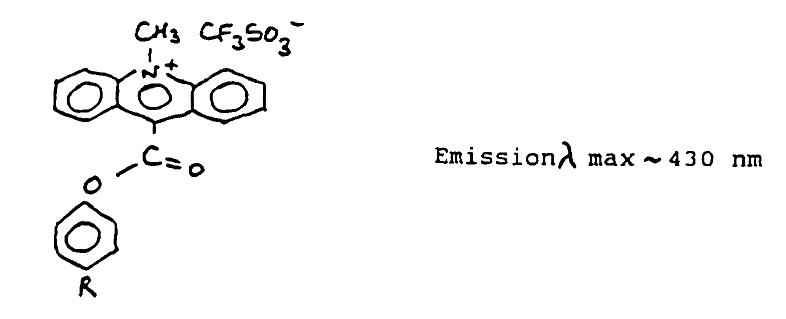
b. 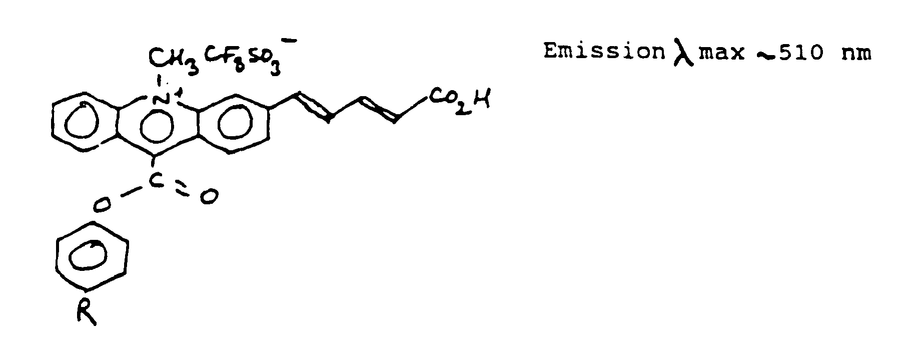
c. 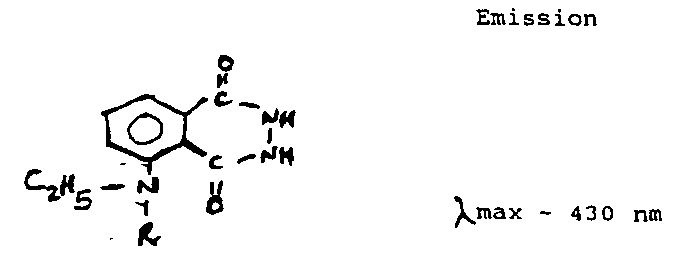
d. 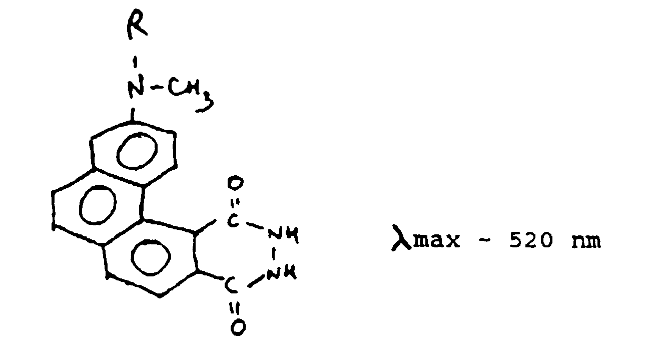
e. 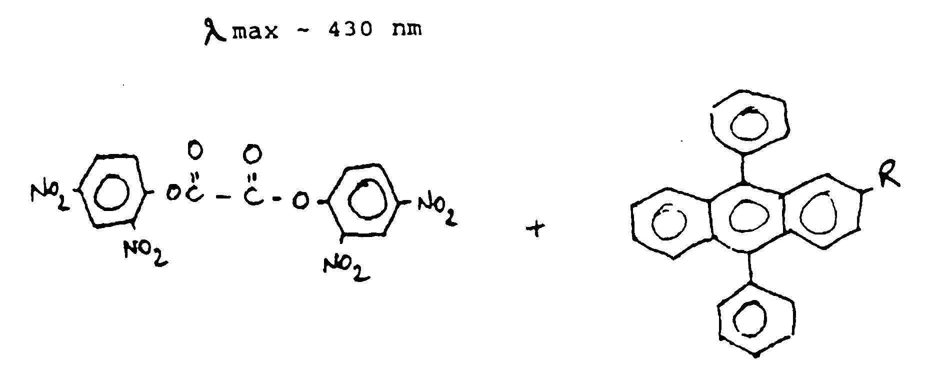
f.

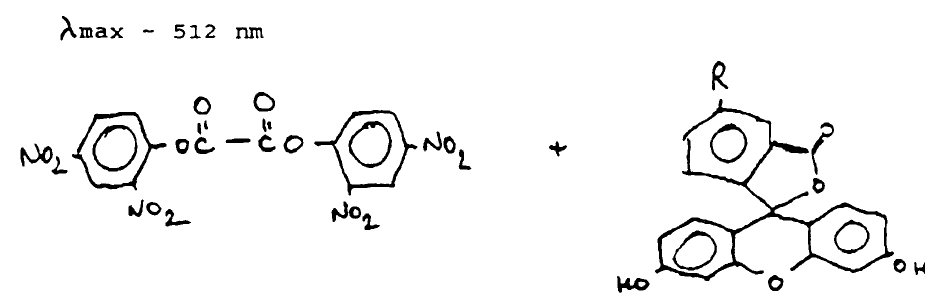

In 2b, the electronic conjugation of the nucleus has been increased so that the emission radiation is of relatively long wavelength.

In each of the acridinium compounds illustrated above, R is selected to allow covalent coupling to a component of the appropriate binding reaction. Appropriate coupling groups are well described but in this example are selected such that the desired kinetic and/or spectroscopic properties of the molecule are maintained and also that the final labelled reagent is still active in terms of its ability to participate in the binding reaction. Such groups include N-hydroxysuccinimide esters, imidate esters, isothiocyanates and other established active group or groups that can give rise to active groups to facilitate coupling to molecules of biological interest. Preferably these groups are linked to the chemiluminescent moiety by an aliphatic chain of appropriate length.

In a further aspect it is possible to also make use of chemiluminescent reactions which involve energy transfer to a fluorescent acceptor molecule. As an example of this it is possible to label an antibody of one specificity with fluorescein and an antibody of another specificity with rhodamine. These antibodies can be used in simultaneous two-site assays and the end-points determined by introduction of a peroxyoxalate chemiluminescence system (hydrogen peroxide/bis-2, 4-dinitrophenyl oxalate). Radiationless energy transfer occurs resulting in the emission of light at two different wavelengths (green-yellow from fluorescein/red from rhodamine), the intensities of the emissions are directly proportional to the amount of the relevant labelled antibody bound in the immunochemical reactions.

### Analyte-binding partners (reagents) and binding reactions

The following schemes represent examples of binding partners or binding reactions that it is possible to use for the determination of concentrations of single analytes which are used currently:
Key:

| | |
|---|---|
| 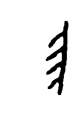 | Solid phase matrix (e.g. coated tube, magnetisable particles) |
| 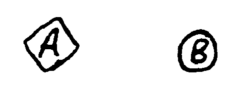 | Different analytes-represented by A and B |
| 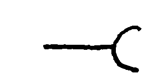 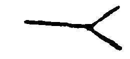 | Antibodies specific for A and B respectively or binding proteins specific for A and B respectively or receptors capable of binding A and B respectively |
| 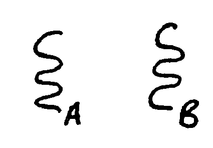 | Oligonucleotide sequences A and B |
| 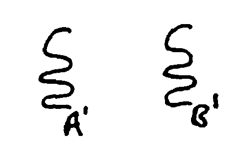 | Complementary oligonucleotide sequences |
| 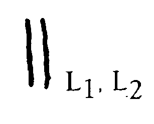 | Binding reagent for double-stranded (recombinant) nucleic acid sequences Chemiluminescent labels exhibiting different reaction kinetics and/or spectroscopic properties respectively. |

1a. Two-Site immunoassays. 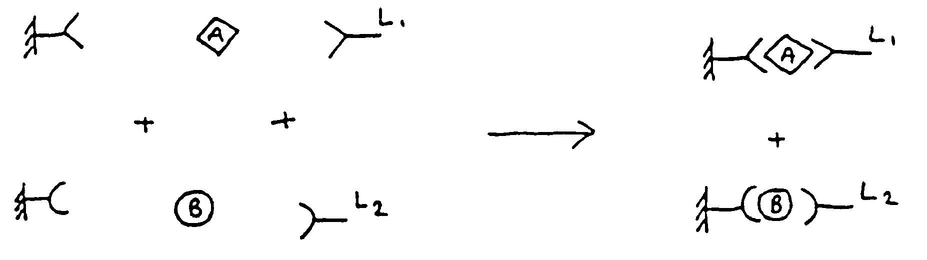
   Antibodies of a given specificity for the analyte recognise different parts of the given analyte to permit formation of the two-site immune complex. Excess concentrations of reagent over analyte are used in two-site systems.
1b Competitive binding immunoassays (labelled antigen) 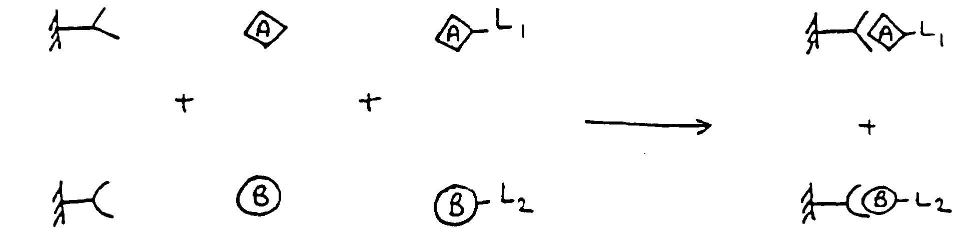
1c Two-site/competitive binding (labelled antigen) combination. 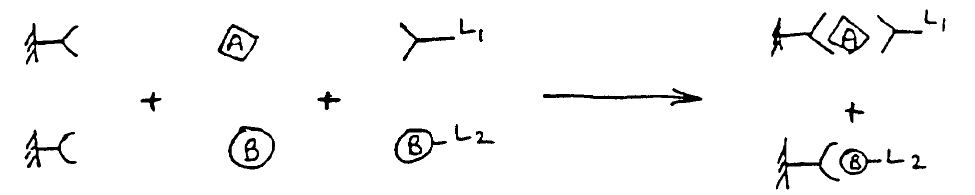
1d Two-site/competitive binding (labelled antibody) combination. 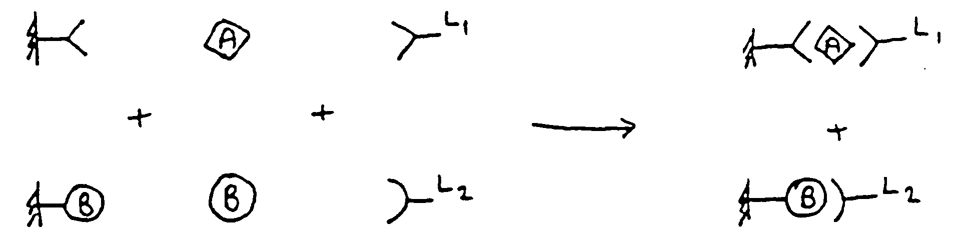
   It should be noted that limiting reagent concentrations are used in competitive binding systems.

2. Oliaonucleotide hybridisation assays 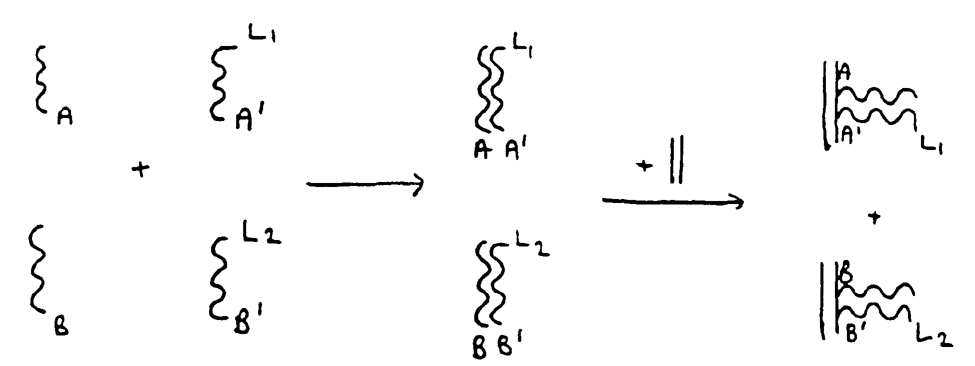
The above are all examples of heterogeneous assay systems in which the analyte/binding-partner complex is isolated from uncomplexed material. Additionally it is possible to apply the disclosed systems to homogeneous assays.

### Instrumentation

Photon counting equipment may be used for the measurement of light intensity. The sensing equipment should be capable of distinguishing th emissions from the distinguishable chemiluminescent reactions.

### 1 A Kinetic discrimination.

As described in the earlier example, the equipment should be capable of recording measurements of light intensity (preferably as photon counts per unit time) within at least two time frames to permit independent measurement of the intensity arising from slow and fast reactions. In many instances there will be overlap between the two signals which is accounted for by appropriate selection of time frames or by mathematical estimation of the overlap.

### 2. Spectroscopic discrimination:

Here it is necessary to measure the intensity of two or more wavelengths simultaneously. This can be achieved, e.g. by use of the necessary number of photomultiplier tubes each fitted with a bandpass interference filter to permit measurement of one signal at the exclusion of others. Alternatively a single photomultiplier tube can be used such that the light emitted from the reaction is first passed through a fast scanning spectrometer or filter/chopper system. Synchronisation of the photomultiplier tube output with the scanning or chopping frequency thus permits independent quantitation of the different wavelengths.

### SPECIFIC EXAMPLE

1. Preparation of labelled antibodies:
a. 4-(2-carbonylethyl) phenyl-10-methyl acridinium-9-carboxylate fluorosulphonate labelled antibodies to human chorionic gonadotrophin (hCG).
   The acridinium label was synthesised as follows: acridinium-9-carboxylic acid (5g) was refluxed with thionyl chloride (15 ml) for 3 hours. The solvent was removed under reduced pressure and the product suspended in anhydrous pyridine (35 ml). Benzyl 4-hydroxyphenylpropanoate (9 nmol) was added and the solution stirred overnight at room temperature. The mixture was subsequently poured into crushed ice/1 M hydrochloric acid (250 ml) and the resulting precipitate filtered, washed with water and dried under reduced pressure. The 4-(2-benzyloxycarbonylethyl)phenyl-9-acridine carboxylate thus obtained was recrystallised from benzene/cyclohexane. 0.46 g of this was dissolved in hydrogen bromide/acetic acid mixture (45/55 w/w, 10 ml) and the solution stirred for 2 h at 50-55°C. The solution was poured into water (100 ml) and the resulting yellow solid, filtered, washed with water and dried under reduced pressure, thence recrystallised from acetonitrile/chloroform to yield 4-(2-carboxyethyl)phenyl-9-acridine carboxylate. N-hydroxysuccinimide (62 mg) was dissolved in dimethylformamide (5 ml) together with 200·mg of the above acridine carboxylate. The mixture was cooled to -20°C and dicyclohexylcarbodiimide (123 mg) added, followed by stirring for 2 h at -20°C, thence overnight at room temperature. One drop of glacial acetic acid was then added and the mixture left for a further 30 min. The dicyclohexylurea was removed by filtration and the material obtained by evaporation of the liquor was recrystallised from benzene/cyclohexane to yield 4-(2-succinimidyloxy carbonylethyl)phenyl-9-acridine carboxylate. The product (234 mg) was dissolved in anhydrous chloroform (25 ml) and methyl fluorosulphonate (0.5 ml) added.The precipitate which formed after stirring at room temperature for 18 hours was filtered and washed with anhydrous benzene to yield 4-(2-succinimidyloxycarbonylethyl)phenyl-10-methylacridinium-9-carboxylate fluorosulphonate. Mouse monoclonal antibodies (50 µg) raised to human chorionic gonadotrophin were dissolved in sodium phosphate buffer (pH 7.4, 0.1 M, 200 µl) containing 0.15 M sodium chloride. A stock solution was made of the acridinium-succinimidyl ester in acetonitrile (0.5 mg/ml) and 10 µl added to the antibody solution with mixing. After incubation at room temperature for 15 min in the dark, a solution of lysine monohydrochloride (100 µl, 10mg/ml) in the above buffer was added and the mixture left for a further 5 min. The mixture was purified on a column of Pharmacia Sephadex G25-M (30 cm x 0.6 cm) equilibrated and eluted with phosphate buffered saline (pH 6.3, 0.1 M, 0.15 M NaCl) containing 0.1% (w/v) bovine serum albumin and 0.05% (w/v) sodium azide. 0.5 ml fractions were collected and the void volume fractions pooled and stored at 4°C.
b. 4-(2-imidylethyl)-2,6-dimethyl-10-methylacridinium-9-carboxylate dichloride labelled antibodies to human alpha-fetoprotein (AFP).

The acridinium label was synthesised as follows: acridinium-9-carboxylic acid (2.5 g) was refluxed with thionyl chloride (10 ml) for 3 hours. The solvent was removed under reduced pressure and the product suspended in anhydrous pyridine (25 ml). 2,6-dimethyl-4-hydroxyphenylpropionitrile (1.3 g) was added and the solution stirred overnight at room temperature. The mixture was subsequently poured into crushed ice/1 M hydrochloric acid (250 ml) and the resulting precipitate filtered, washed with water and dried under reduced pressure. The 4-(2-cyanoethyl) -2,6-dimethylphenylacridinium carboxylate thus obtained was dissolved in anhydrous chloroform (15 ml) and methyltrifluoromethylsulphonate (0.5 ml) added. The precipitate which formed after stirring overnight at room temperature and addition of diethylether was filtered off and washed with anhydrous benzene to yield 4-(2-cyanoethyl)-2,6-dimethyl-phenyl-10-methyl-9-acridiniumcarboxylate trifluoromethylsulphonate which was subsequently dissolved (69 mg, 10 ml) in anhydrous methanol. HC1 gas was bubbled through the solution under nitrogen, kept at ice temperature for 2 hours and left to stand for a further 1 hour. The crystals formed were filtered under an atmosphere of dry nitrogen and washed with anhydrous methanol to yield 4-(2-methyloxyimidylethyl)-2,6-dimethylphenyl-10-methylacridinium-9-carboxylate dichloride. Mouse monoclonal antibodies (50 µg) raised to human alpha-fetoprotein were dissolved in sodium borate buffer (pH 9.5, 0.1 M, 200 µl) containing 0.15 M sodium chloride. A stock solution was made of acridinium imidoester in acetonitrile (0.5 mg/ml) and 190 µl added to the antibody solution with mixing. After incubation at room temperature for 30 min in the dark, a solution of lysine monohydrochloride (100 µl, 10mg/ml) in the above buffer was added and the mixture left for a further 15 mins. The mixture was purified as described above.
2. Preparation of solid-phase antibodies
Monoclonal antibodies to human chorionic gonadotrophin and alpha-fetoprotein recognising distinct epitopes to those recognised by the labelled antibodies were coupled to paramagnetic particles using published methods.
3. "Simultaneous" immunochemiluminometric assay for human chorionic gonadotrophin and human alpha-fetoprotein.
Solid phase antibody suspensions (800 µg/ml) were mixed in equal volumes. Labelled antibody solutions (10 ng/ml anti-hCG, 50 ng/ml anti-AFP) were mixed in equal volumes. Diluent buffer was same as antibody purification buffer (above). Standard mixtures for calibration consisted of solutions containing known concentrations of hCG and AFP in horse serum. 50 µl of patient serum sample were dispensed in duplicate into 12 x 75 mm polystyrene test tube. Standard tubes were set up in duplicate using 50 µl of the appropriate standard 100 µl of the labelled antibody mixture were added followed by 100 µl of the solid-phase antibody mixture. The tubes were mixed and set aside for 1 hour at room temperature. lml of wash solution (1.76 g/l sodium dihydrogen orthophosphate, 0.15M sodium chloride, 0.5% (W/v) sodiumazide, 0.5% bovine serum albumin, 1% (v/v) Triton X-100) was added and the tubes placed in a magnetic rack to facilitate sedimentation of the solid phase. The supernatants were decanted to waste and a further 1 ml of wash buffer added followed by mixing of the tube contents. A further sedimentation/decantation step was performed and the tubes placed in a luminometer.
4. Measurement of light intensity.
Measurements of light emission were made in a Ciba Corning Magic Lite Analyzer using procedures recommended by the Manufacturer. Manipulation of the software enabled distinct, sequential integration of light intensity with respect to time. Separate integrations were performed in the ranges 0 - 1 and 1 - 2 seconds corresponding to light emission from the hCG and AFP antibodies respectively and hence being proportional to the concentrations of hCG and AFP in the sample. Since some overlap existed due to interference with the hCG signal by the AFP signal, correction was necessary. This was achieved by estimating the AFP contribution to the 0 - 1 second integration using the previously determined relationship of the light intensity at 2 seconds to the integral between 0 -1 seconds, as can be seen in Figure 2

| [hCG] U/L | Photon Counts | [AFP] kU/L | Photon Counts | Overlap | Corrected hCG Photon Counts |
|---|---|---|---|---|---|
| 250 | 1.22 × 10⁶ | 250 | 1.14 × 10⁵ | 3.35 × 10⁴ | 1.19 × 10⁶ |
| 125 | 8.11 × 10⁵ | 125 | 7.4 × 10⁴ | 2.18 × 10⁴ | 7.89 × 10⁵ |
| 62.5 | 4.66 × 10⁵ | 62.5 | 4.2 × 10⁴ | 1.24 × 10⁴ | 4.54 × 10⁵ |
| 31.3 | 2.63 × 10⁵ | 31.3 | 2.3 × 10⁴ | 6.76 × 10³ | 2.56 × 10⁵ |
| 15.6 | 1.33 × 10⁵ | 5.6 | 1.3 × 10⁴ | 3.82 × 10³ | 1.29 × 10⁵ |
| 0 | 3.37 × 10³ | 0 | 6.6 × 10² | 1.94 × 10² | 3.18 × 10³ |

r=3.40
When [hCG] >> [AFP] the residual hCG signal during the second time window made a significant contribution hence perturbing measurement of x. This was not a problem in practice and could be minimised by relative assay optimisation e.g. by manipulation of specific activities of the labelled antibodies.
In situations where accuracy is required even at extreme relative concentrations mutual overlap is accounted for by the use of more complex software capable of iterative or simulatenous equation calculation.
Where overlap is a serious problem alternative labels must be sought that exhibit greater differences in their relative kinetic properties.

### REFERENCES

1. I. Hemmilä, S. Holttinen, K. Pettersson, T. Lövgren Clinical Chemistry 33 (1987) 2281
2. B. Edwards, A. Sparks, J.C. Voyta, I. Bronstein Journal of Bioluminescence and Chemiluminescence 5 (1990) 1
3. K.V. Wood, Y.A- Lam, W.D. McElroy, H.H. Seliger Journal of Bioluminescence and Chemiluminescence 4 (1989) 31
4. F. McCapra, Accounts of Chemical Research 9 (1976) 201
5. F. McCapra, et al, Journal of Bioluminesence and Chemiluminescence 4 (1989) 51
6. B. Edwards, A. Sparks, J.C. Voyta, I. Bronstein Journal of Bioluminescence and Chemiluminescence 5 (1990) 1
7. M.M. Rauhut, L.J. Bollyky, B.G. Roberts, et al, Journal of the American Chemical Society 89 (1967) 6515.
8. K.D. Gundermann, Topics in Current Chemistry 46 (1974) 61.
9. U.S. Patent No. 3689391 (Ullman)
10. U.S. Patent No. 4277437 (Maggio).
11. U.S. Patent No. 3817837 (Rubenstein)

## Claims (Claims for the following Contracting State(s): AT, BE, CH, DE, DK, FR, GB, IT, LI, LU, NL, SE)

1. A method for the assay, detection, quantification, location or analysis of a sample containing at least two substances of interest comprising the steps of
i) reacting said sample with a mixture comprising at least a first reagent and a second reagent that form at least a first complex and a second complex wherein said first complex comprises one of said substances or a respective associated substance and said first reagent, and wherein said second complex comprises another said substance and said second reagent, wherein at least said first reagent and second reagent each comprise
a) a binding partner for binding or otherwise linking with one or more of said substances, and
b) a different chemiluminescent molecule exhibiting distinguishable emission characteristics, wherein said chemiluminescent molecule is chemically or physically coupled to said binding partner;
ii) subsequently treating said sample comprising said first and second complexes to cause a first and second chemiluminescent reaction to occur, wherein said chemiluminescent reactions are simultaneously triggered, and
iii) observing, sensing, measuring and/or recording the emissions of each of said chemiluminescent reactions.

2. The method of claim 1, wherein the emission characteristics of each of said chemiluminescent reactions are distinguishable in terms of the variation of light emission or radiation intensity with the time of the emissions.

3. The method according to claim 2, wherein the intensity of the radiation emitted from said sample is observed, sensed, measured and/or recorded over two different time intervals wherein said time intervals, may overlap.

4. The method according to claim 1, wherein each of said chemiluminescent reactions emits radiation in a respective different spectral range.

5. The method according to claim 1, involving at least three chemiluminescent reactions, wherein at least two of said chemiluminescent reactions are distinguishable in terms of the spectral emission characteristics and wherein at least two of said chemiluminescent reactions are distinguishable in terms of the variation of light or radiation intensity with time.

6. The method according to claim 4 or 5, wherein the emissions are filtered by respective filtering means responsive to radiation in said different spectral ranges and the filtered intensities of the emissions are observed, sensed, measured and/or recorded.

7. The method according to claim 1 or any claim dependent thereon, wherein at least one of said first and second complexes is made up by a reagent bound to a substance by one of an immunoassay binding reaction, a protein binding reaction, a nucleic acid hybridization and a receptor binding reaction.

8. The method according to any preceding claim, wherein each of said chemiluminescent reactions includes a respective component or reagent that is a structural variant of the or each component or reagent associated with the or each other chemiluminescent reactions.

9. The method according to any of the preceding claims, wherein one of the components in at least one of said chemiluminescent reactions is based on acridinium compounds, or structural variants thereof.

10. The method according to claim 9, wherein one of the components in at least one of said chemiluminescent reactions is an acridinium salt alkylated at the ring nitrogen.

11. The method according to claim 10, wherein one of said components in at least one of said chemiluminescent reactions is an acridinium phenylester wherein the ester is formed at position 9 of the acridine nucleus.

12. The method according to claim 11 wherein one of said chemiluminescent reactions includes an acridinium phenylester wherein the phenyl moiety is substituted with electron withdrawing groups to yield a chemiluminescent reaction in which the emission of light occurs over a relatively short period, and wherein another of said chemiluminescent reactions includes an acridinium phenylester, wherein the phenyl moiety is substituted with electron donating groups to yield a chemiluminescent reaction in which the emission of light occurs over a relatively long period.

13. The method according to claim 10, wherein one of said chemiluminescent reactions includes an acridinium salt which, upon triggering said reaction, emits radiation at a relatively short wavelength, and wherein another of said chemiluminescent reactions includes an acridinium salt, wherein the electronic conjugation of the acridine nucleus is increased whereby, upon triggering of said another reaction, said acridinium salt emits at a relatively long wavelength.

14. The method according to claim 13, wherein said one chemiluminescent reaction emits radiation of wavelength in the range of from 400 to 500 nm and said other chemiluminescent reaction emits radiation of wavelength in the range of from 500 to 700 nm.

15. The method according to claim 9 or any claim dependent thereon, wherein the acridinium compound is modified or further derivatized to permit covalent coupling to a molecule of biological interest.

16. The method according to any of claims 1 to 8, wherein one of the components in at least one of said chemiluminescent reactions is based upon one of the following compounds:
i) phthalhydrazides and related compounds;
ii) dioxetanes, dioxetanones and related compounds; and
iii) bis-oxalate esters, related compounds and associated acceptor partners where required.

17. A luminescent reagent which comprises a mixture of at least two reagents, wherein said reagents each comprise
a) a binding partner for binding or otherwise linking with respective substances of interest; and
b) a different chemiluminescent molecule exhibiting distinguishable emission characteristics, chemically or physically coupled to the binding partner.

18. The luminescent reagent according to claim 17, wherein each of said chemiluminescent emissions are distinguishable from each other in terms of the variation of light intensity with time of the emissions.

19. The luminescent reagent according to claim 17, wherein each of said chemiluminescent emissions emits radiation in a different spectral range.

20. The luminescent reagent according to claim 17 or claim 18, wherein at least one of said reagents is capable of binding or otherwise linking in a reaction selected from the group consisting of an immunoassay binding reaction, a protein binding reaction, a nucleic acid hybridization reaction and a receptor binding reaction.

21. The luminescent reagent according to any one of claims 17, 18, 19 and 20, wherein said chemiluminescent molecules are structural variants of the or each other chemiluminescent molecule.

22. The luminescent reagent according to any of claims 17, 18, 19 and 20, wherein one of the chemiluminescent molecules capable of taking part in at least one respective chemiluminecent reaction is based on acridinium compounds or structural variants thereof.

23. The luminescent reagent according to claim 22, wherein one of the chemiluminescent molecules in at least one of said chemiluminescent reactions is acridinium salt alkylated at the ring nitrogen.

24. The luminescent reagent according to claim 22 wherein one of said chemiluminescent molecules in at least one of said chemiluminescent reactions is an acridinium phenylester wherein the ester is formed at position 9 of the acridine nucleus.

25. The luminescent reagent according to claim 24 wherein one of said chemiluminescent reactions includes an acridinium phenylester wherein the phenyl moiety is substituted with electron withdrawing groups to yield a chemiluminescent reaction in which the emission of light occurs over a relatively short period, and wherein another of said chemiluminescent reactions includes an acridinium phenylester, wherein the phenyl moiety is substituted with electron donating groups to yield a chemiluminescent reaction in which the emission of light occurs over a relatively long period.

26. The luminescent reagent according to claim 22 wherein one of said chemiluminescent reactions includes an acridinium salt which, upon triggering of said reaction, emits radiation at a relatively short wavelength and wherein another of said chemiluminescent reactions includes an acridinium salt, wherein the electronic conjugation of the acridine nucleus is increased whereby, upon triggering of said another reaction, said acridinium salt emits radiation at a relatively long wavelength.

27. The luminescent reagent according to claim 26 wherein said one chemiluminescent reaction emits radiation of wavelength in the range of from 400 nm to 500 nm and said other chemiluminescent reaction emits radiation in the range of from 500 to 700 nm.

28. A luminescent reagent according to claim 22 or any claim dependent thereon, wherein the acridinium compound is modified or further derivatized to permit covalent coupling to a molecule of biological interest.

29. A luminescent reagent according to any of claims 17, 18, 19 and 20, wherein said chemiluminescent molecules are capable of taking part in respective chemiluminescent reactions , wherein at least one of said chemiluminescent reactions is based upon one of the following compounds:
i) phthalhydrazides and related compounds;
ii) dioxetanes, dioxetanones and related compounds; and
iii) bis-oxalate esters, related compounds and associated acceptor partners where required.

## Claims (Claims for the following Contracting State(s): ES)

1. A method for the assay, detection, quantification, location or analysis of a sample containing at least two substances of interest comprising the steps of
i) reacting said sample with a mixture comprising at least a first reagent and a second reagent that form at least a first complex and a second complex wherein said first complex comprises one of said substances or a respective associated substance and said first reagent, and wherein said second complex comprises another said substance and said second reagent, wherein at least said first reagent and second reagent each comprise
a) a binding partner for binding or otherwise linking with one or more of said substances, and
b) a different chemiluminescent molecule exhibiting distinguishable emission characteristics, wherein said chemiluminescent molecule is chemically or physically coupled to said binding partner;
ii) subsequently treating said sample comprising said first and second complexes to cause a first and second chemiluminescent reaction to occur, wherein said chemiluminescent reactions are simultaneously triggered, and
iii) observing, sensing, measuring and/or recording the emissions of each of said chemiluminescent reactions.

2. The method of claim 1, wherein the emission characteristics of each of said chemiluminescent reactions are distinguishable in terms of the variation of light emission or radiation intensity with the time of the emissions.

3. The method according to claim 2, wherein the intensity of the radiation emitted from said sample is observed, sensed, measured and/or recorded over two different time intervals wherein said time intervals, may overlap.

4. The method according to claim 1 wherein each of said chemiluminescent reactions emits radiation in a respective different spectral range.

5. The method according to claim 1, involving at least three chemiluminescent reactions, wherein at least two of said chemiluminescent reactions are distinguishable in terms of the spectral emission characteristics and wherein at least two of said chemiluminescent reactions are distinguishable in terms of the variation of light or radiation intensity with time.

6. The method according to claim 4 or 5, wherein the emissions are filtered by respective filtering means responsive to radiation in said different spectral ranges and the filtered intensities of the emissions are observed, sensed, measured and/or recorded.

7. The method according to claim 1 or any claim dependent thereon, wherein at least one of said first and second complexes is made up by a reagent bound to a substance by one of an immunoassay binding reaction, a protein binding reaction, a nucleic acid hybridization and a receptor binding reaction.

8. The method according to any preceding claim, wherein each of said chemiluminescent reactions includes a respective component or reagent that is a structural variant of the or each component or reagent associated with the or each other chemiluminescent reactions.

9. The method according to any of the preceding claims, wherein one of the components in at least one of said chemiluminescent reactions is based on acridinium compounds, or structural variants thereof.

10. The method according to claim .9, wherein one of the components in at least one of said luminescent reactions is an acridinium salt alkylated at the ring nitrogen.

11. The method according to claim 10, wherein one of said components in at least one of said chemiluminescent reactions is an acridinium phenylester wherein the ester is formed at position 9 of the acridine nucleus.

12. The method according to claim 11 wherein one of said chemiluminescent reactions includes an acridinium phenylester wherein the phenyl moiety is substituted with electron withdrawing groups to yield a chemiluminescent reaction in which the emission of light occurs over a relatively short period, and wherein another of said chemiluminescent reactions includes an acridinium phenylester, wherein the phenyl moiety is substituted with electron donating groups to yield a chemiluminescent reaction in which the emission of light occurs over a relatively long period.

13. The method according to claim 10, wherein one of said chemiluminescent reactions includes an acridinium salt which, upon triggering said reaction, emits radiation at a relatively short wavelength, and wherein another of said chemiluminescent reactions includes an acridinium salt, wherein the electronic conjugation of the acridine nucleus is increased whereby, upon triggering of said another reaction, said acridinium salt emits at a relatively long wavelength.

14. The method according to claim 13, wherein said one chemiluminescent reaction emits radiation of wavelength in the range of from 400 to 500 nm and said other chemiluminescent reaction emits radiation of wavelength in the range of from 500 to 700 nm.

15. The method according to claim 9 or any claim dependent thereon, wherein the acridinium compound is modified or further derivatized to permit covalent coupling to a molecule of biological interest.

16. The method according to any of claims 1 to 8, wherein one of the components in at least one of said chemiluminescent reactions is based upon one of the following compounds:
i) phthalhydrazides and related compounds;
ii) dioxetanes, dioxetanones and related compounds; and
iii) bis-oxalate esters, related compounds and associated acceptor partners where required.

## Patentansprüche (Patentansprüche für folgende(n) Vertragsstaat(en): AT, BE, CH, DE, DK, FR, GB, IT, LI, LU, NL, SE)

1. Verfahren zur Untersuchung, zum Nachweis, zur Quantifizierung, zum Auffinden oder zur Analyse einer Probe, die mindestens zwei Substanzen von Interesse enthält, umfassend die Schritte:
i) Zur-Reaktion-bringen der Probe mit einem Gemisch, das mindestens ein erstes Reagenz und ein zweites Reagenz umfasst, die mindestens einen ersten und einen zweiten Komplex bilden, wobei der erste Komplex eine der Substanzen oder eine entsprechende damit verbundene Substanz und das erste Reagenz umfasst, und wobei der zweite Komplex eine andere Substanz und das zweite Reagenz umfasst, wobei mindestens das erste Reagenz und das zweite Reagenz umfassen
a) einen Bindungspartner für die Bindung oder eine andere Art der Kopplung mit einer oder mehreren der Substanzen, und
b) ein anderes chemilumineszentes Molekül, das unterscheidbare Emissionsmerkmale aufweist, wobei das chemilumineszente Molekül chemisch oder physikalisch an den Bindungspartner gekoppelt ist;
ii) anschließende Behandlung der den ersten und zweiten Komplex umfassenden Probe zum Hervorrufen einer ersten und zweiten chemilumineszenten Reaktion, wobei die chemilumineszenten Reaktionen gleichzeitig ausgelöst werden, und
iii) Beobachten, Erfassen, Messen und/oder Aufzeichnen der Emissionen jeder der chemilumineszenten Reaktionen.

2. Verfahren nach Anspruch 1, wobei die Emissionsmerkmale jeder der chemilumineszenten Reaktionen auf Grund der Änderungen der Intensität der Lichtemission oder der Intensität der Strahlung mit der Emissionszeit unterscheidbar sind.

3. Verfahren nach Anspruch 2, wobei die Intensität der von der Probe emittierten Strahlung über zwei unterschiedliche Zeitintervalle, die sich überlappen können, beobachtet, erfasst, gemessen und/oder aufgezeichnet wird.

4. Verfahren nach Anspruch 1, wobei jede einzelne chemilumineszente Reaktion eine Strahlung in einem jeweils unterschiedlichen Spektralbereich emittiert.

5. Verfahren nach Anspruch 1, bei dem wenigstens drei chemilumineszente Reaktionen auftreten, von denen wenigstens zwei der chemilumineszenten Reaktionen hinsichtlich der spektralen Emissionsmerkmale und wenigstens zwei auf Grund der Änderung der Intensität des Lichtes oder der Intensität der Strahlung mit der Zeit unterscheidbar sind.

6. Verfahren nach Anspruch 4 oder 5, bei dem die Emissionen mittels entsprechender Filter gefiltert werden, die auf die Strahlung in den verschiedenen Spektralbereichen ansprechen, und die gefilterten Intensitäten der Emissionen beobachtet, erfasst, gemessen und/oder aufgezeichnet werden.

7. Verfahren nach Anspruch 1 oder einem von diesem abhängigen Anspruch, bei dem wenigstens einer der ersten und zweiten Komplexe aus einem Reagenz besteht, das an eine Substanz durch eine Immunoassay-Bindungsreaktion, eine Protein-Bindungsreaktion, eine Nucleinsäure-Hybridisierungs- und eine Rezeptorreaktion gebunden ist.

8. Verfahren nach einem der vorhergehenden Ansprüche, bei dem jede einzelne chemilumineszente Reaktion eine entsprechende Komponente oder ein Reagenz enthält, das eine strukturelle Variante des oder jedes Bestandteiles oder Reagenzes ist, das der oder jeder anderen chemilumineszenten Reaktion zugeordnet ist.

9. Verfahren nach einem der vorangehenden Ansprüche, bei dem eine der Komponenten in wenigstens einer der chemilumineszenten Reaktionen auf Acridinium-Verbindungen oder deren strukturellen Varianten basiert.

10. Verfahren nach Anspruch 9, bei dem eine der Komponenten in wenigstens einer der chemilumineszenten Reaktionen ein Acridiniumsalz ist, das am Ringstickstoff alkyliert ist.

11. Verfahren nach Anspruch 10, bei dem eine der Komponenten in wenigstens einer der chemilumineszenten Reaktionen ein Acridiniumphenylester ist, wobei der Ester an Position 9 des Acridinkerns gebildet wird.

12. Verfahren nach Anspruch 11, wobei eine der chemilumineszenten Reaktionen einen Acridiniumphenylester einschließt, dessen Phenylanteil mit Elektronen abziehenden Gruppen substituiert ist, um eine chemilumineszente Reaktion hervorzurufen, deren Lichtemission über einen verhältnismäßig kurzen Zeitraum auftritt, und eine andere chemilumineszente Reaktion einen Acridiniumphenylester umfasst, dessen Phenylanteil mit Elektronen abgebenden Gruppen substituiert ist, um eine chemilumineszente Reaktion zu ergeben, deren Lichtemission über einen verhältnismäßig langen Zeitraum auftritt.

13. Verfahren nach Anspruch 10, bei dem eine der chemilumineszenten Reaktionen ein Acridiniumsalz einschließt, das beim Einleiten der Reaktion eine Strahlung mit einer verhältnismäßig kurzen Wellenlänge ausstrahlt, und bei dem eine andere chemilumineszente Reaktion ein Acridiniumsalz einschließt, bei dem die elektronische Konjugation des Acridinkerns vergrößert ist, wobei beim Einleiten dieser anderen Reaktion das Acridiniumsalz eine Strahlung mit einer verhältnismäßig langen Wellenlänge emittiert.

14. Verfahren nach Anspruch 13, bei dem die eine chemilumineszente Reaktion eine Strahlung mit einer Wellenlänge im Bereich von 400 bis 500 nm und die andere chemilumineszente Reaktion eine Strahlung mit einer Wellenlänge im Bereich von 500 bis 700 nm ausstrahlt.

15. Verfahren nach Anspruch 9 oder einem von diesem abhängigen Anspruch, bei dem die Acridiniumverbindung modifiziert oder weiter derivatisiert ist, um kovalente Bindungen an ein Molekül von biologischem Interesse zu ermöglichen.

16. Verfahren nach einem der Ansprüche 1 bis 8, bei dem eine der Verbindungen in wenigstens einer der chemilumineszenten Reaktionen auf einer der folgenden Verbindungen basiert:
i) Phthalhydrazide und verwandte Verbindungen,
ii) Dioxetane, Dioxetanone und verwandte Verbindungen; und
iii) Bis-oxalsäureester, verwandte Verbindungen und falls erforderlich, assoziierte Akzeptorpartner.

17. Lumineszentes Reagenz, das ein Gemisch von wenigstens zwei Reagenzien umfasst, wobei jedes der Reagenzien umfasst
a) einen Bindungspartner für die Bindung oder sonstige Kopplung an entsprechende Substanzen von Interesse; und
b) ein anderes chemilumineszentes Molekül, das unterscheidbare Emissionsmerkmale aufweist, wobei das chemilumineszente Molekül chemisch oder physikalisch an den Bindungspartner gekoppelt ist.

18. Lumineszentes Reagenz nach Anspruch 17, bei dem jede der chemilumineszenten Emissionen von jeder anderen auf Grund der Änderung der Lichtintensität mit der Emissionszeit unterscheidbar ist.

19. Lumineszentes Reagenz nach Anspruch 17, bei dem jede der chemilumineszenten Emissionen eine Strahlung in einem unterschiedlichen Spektralbereich emittiert.

20. Lumineszentes Reagenz nach Anspruch 17 oder 18, bei dem wenigstens eine der Reagenzien in der Lage ist, durch eine Reaktion ausgewählt aus der Gruppe bestehend aus einer Immunoassay-Bindungsreaktion, einer Proteinbindungsreaktion, einer Nucleinsäure-Hybridisierungsreaktion und einer Rezeptor-Bindungsreaktion zu binden oder in anderer Weise zu verknüpfen.

21. Lumineszentes Reagenz nach einem der Ansprüche 17, 18, 19 und 20, bei dem die chemilumineszenten Moleküle strukturelle Varianten des oder jedes anderen chemilumineszenten Moleküls sind.

22. Lumineszentes Reagenz nach einem der Ansprüche 17, 18, 19 und 20, bei dem eines der chemilumineszenten Moleküle, das in der Lage ist, an wenigstens einer der chemilumineszenten Reaktionen teilzunehmen, auf Acridiniumverbindungen oder deren strukturellen Varianten basiert.

23. Lumineszentes Reagenz nach Anspruch 22, bei dem eines der chemilumineszenten Moleküle in wenigstens einer der chemilumineszenten Reaktionen ein Acridiniumsalz ist, das am Ringstickstoff alkyliert ist.

24. Lumineszentes Reagenz nach Anspruch 22, bei dem eines der chemilumineszenten Moleküle in wenigstens einer der chemilumineszenten Reaktionen ein Acridiniumphenylester ist, wobei der Ester an Position 9 des Acridinkerns gebildet wird.

25. Lumineszentes Reagenz nach Anspruch 24, bei dem eine der chemilumineszenten Reaktionen einen Acridiniumphenylester einschließt, dessen Phenylanteil mit Elektronen abziehenden Gruppen substituiert ist, um eine chemilumineszente Reaktion zu erzielen, deren Lichtemission über einen relativ kurzen Zeitraum auftritt, und eine andere chemilumineszente Reaktion einen Acridiumphenylester umfasst, dessen Phenylanteil mit Elektronen abgebenden Gruppen substituiert ist, um eine chemilumineszente Reaktion zu erzielen, deren Lichtemission über einen relativ langen Zeitraum auftritt.

26. Lumineszentes Reagenz nach Anspruch 22, bei dem eine der chemilumineszenten Reaktionen ein Acridiniumsalz einschließt, das beim Einleiten der Reaktion eine Strahlung mit einer verhältnismäßig kurzen Wellenlänge ausstrahlt, und bei dem eine andere chemilumineszente Reaktion ein Acridiniumsalz einschließt, bei dem die elektronische Konjugation des Acridinkerns vergrößert ist, wobei beim Einleiten dieser anderen Reaktion das Acridiniumsalz eine Strahlung mit verhältnismäßig langer Wellenlänge emittiert.

27. Lumineszentes Reagenz nach Anspruch 26, bei dem die eine chemilumineszente Reaktion eine Strahlung mit einer Wellenlänge im Bereich von 400 nm bis 500 nm und die andere chemilumineszente Reaktion eine Strahlung im Bereich von 500 nm bis 700 nm emittiert.

28. Lumineszentes Reagenz nach Anspruch 22 oder einem von diesem abhängigen Anspruch, bei dem die Acridiniumverbindung modifiziert oder weiter derivatisiert ist, um kovalente Bindungen an ein Molekül von biologischem Interesse zu ermöglichen.

29. Lumineszentes Reagenz nach einem der Ansprüche 17, 18, 19 und 20, wobei die chemilumineszenten Moleküle in der Lage sind, an den chemilumineszenten Reaktionen teilzunehmen, wobei wenigstens eine der chemilumineszenten Reaktionen auf einer der folgenden Verbindungen basiert:
i) Phthalhydrazide und verwandte Verbindungen,
ii) Dioxetane, Dioxetanone und verwandte Verbindungen; und
iii) Bis-oxalsäureester, verwandte Verbindungen und, falls erforderlich, assoziierte Akzeptorpartner.

## Patentansprüche (Patentansprüche für folgende(n) Vertragsstaat(en): ES)

1. Verfahren zur Untersuchung, zum Nachweis, zur Quantifizierung, zum Auffinden oder zur Analyse einer Probe, die mindestens zwei Substanzen von Interesse enthält, umfassend die Schritte:
i) Zur-Reaktion-bringen der Probe mit einem Gemisch, das mindestens ein erstes Reagenz und ein zweites Reagenz umfasst, die mindestens einen ersten und einen zweiten Komplex bilden, wobei der erste Komplex eine der Substanzen oder eine entsprechende damit verbundene Substanz und das erste Reagenz umfasst, und wobei der zweite Komplex eine andere Substanz und das zweite Reagenz umfasst, wobei mindestens das erste Reagenz und das zweite Reagenz umfassen
a) einen Bindungspartner für die Bindung oder eine andere Art der Kopplung mit einer oder mehreren der Substanzen, und
b) ein anderes chemilumineszentes Molekül, das unterscheidbare Emissionsmerkmale aufweist, wobei das chemilumineszente Molekül chemisch oder physikalisch an den Bindungspartner gekoppelt ist;
ii) anschließende Behandlung der den ersten und zweiten Komplex umfassenden Probe zum Hervorrufen einer ersten und zweiten chemilumineszenten Reaktion, wobei die chemilumineszenten Reaktionen gleichzeitig ausgelöst werden, und
iii) Beobachten, Erfassen, Messen und/oder Aufzeichnen der Emissionen jeder der chemilumineszenten Reaktionen.

2. Verfahren nach Anspruch 1, wobei die Emissionsmerkmale jeder der chemilumineszenten Reaktionen auf Grund der Änderungen der Intensität der Lichtemission oder der Intensität der Strahlung mit der Emissionszeit unterscheidbar sind.

3. Verfahren nach Anspruch 2, wobei die Intensität der von der Probe emittierten Strahlung über zwei unterschiedliche Zeitintervalle, die sich überlappen können, beobachtet, erfasst, gemessen und/oder aufgezeichnet wird.

4. Verfahren nach Anspruch 1, wobei jede einzelne chemilumineszente Reaktion eine Strahlung in einem jeweils unterschiedlichen Spektralbereich emittiert.

5. Verfahren nach Anspruch 1, bei dem wenigstens drei chemilumineszente Reaktionen auftreten, von denen wenigstens zwei der chemilumineszenten Reaktionen hinsichtlich der spektralen Emissionsmerkmale und wenigstens zwei auf Grund der Änderung der Intensität des Lichtes oder der Intensität der Strahlung mit der Zeit unterscheidbar sind.

6. Verfahren nach Anspruch 4 oder 5, bei dem die Emissionen mittels entsprechender Filter gefiltert werden, die auf die Strahlung in den verschiedenen Spektralbereichen ansprechen, und die gefilterten Intensitäten der Emissionen beobachtet, erfasst, gemessen und/oder aufgezeichnet werden.

7. Verfahren nach Anspruch 1 oder einem von diesem abhängigen Anspruch, bei dem wenigstens einer der ersten und zweiten Komplexe aus einem Reagenz besteht, das an eine Substanz durch eine Immunoassay-Bindungsreaktion, eine Protein-Bindungsreaktion, eine Nucleinsäure-Hybridisierungs- und eine Rezeptorreaktion gebunden ist.

8. Verfahren nach einem der vorhergehenden Ansprüche, bei dem jede einzelne chemilumineszente Reaktion eine entsprechende Komponente oder ein Reagenz enthält, das eine strukturelle Variante des oder jedes Bestandteiles oder Reagenzes ist, das der oder jeder anderen chemilumineszenten Reaktion zugeordnet ist.

9. Verfahren nach einem der vorangehenden Ansprüche, bei dem eine der Komponenten in wenigstens einer der chemilumineszenten Reaktionen auf Acridinium-Verbindungen oder deren strukturellen Varianten basiert.

10. Verfahren nach Anspruch 9, bei dem eine der Komponenten in wenigstens einer der chemilumineszenten Reaktionen ein Acridiniumsalz ist, das am Ringstickstoff alkyliert ist.

11. Verfahren nach Anspruch 10, bei dem eine der Komponenten in wenigstens einer der chemilumineszenten Reaktionen ein Acridiniumphenylester ist, wobei der Ester an Position 9 des Acridinkerns gebildet wird.

12. Verfahren nach Anspruch 11, wobei eine der chemilumineszenten Reaktionen einen Acridiniumphenylester einschließt, dessen Phenylanteil mit Elektronen abziehenden Gruppen substituiert ist, um eine chemilumineszente Reaktion hervorzurufen, deren Lichtemission über einen verhältnismäßig kurzen Zeitraum auftritt, und eine andere chemilumineszente Reaktion einen Acridiniumphenylester umfasst, dessen Phenylanteil mit Elektronen abgebenden Gruppen substituiert ist, um eine chemilumineszente Reaktion zu ergeben, deren Lichtemission über einen verhältnismäßig langen Zeitraum auftritt.

13. Verfahren nach Anspruch 10, bei dem eine der chemilumineszenten Reaktionen ein Acridiniumsalz einschließt, das beim Einleiten der Reaktion eine Strahlung mit einer verhältnismäßig kurzen Wellenlänge ausstrahlt, und bei dem eine andere chemilumineszente Reaktion ein Acridiniumsalz einschließt, bei dem die elektronische Konjugation des Acridinkerns vergrößert ist, wobei beim Einleiten dieser anderen Reaktion das Acridiniumsalz eine Strahlung mit einer verhältnismäßig langen Wellenlänge emittiert.

14. Verfahren nach Anspruch 13, bei dem die eine chemilumineszente Reaktion eine Strahlung mit einer Wellenlänge im Bereich von 400 bis 500 nm und die andere chemilumineszente Reaktion eine Strahlung mit einer Wellenlänge im Bereich von 500 bis 700 nm ausstrahlt.

15. Verfahren nach Anspruch 9 oder einem von diesem abhängigen Anspruch, bei dem die Acridiniumverbindung modifiziert oder weiter derivatisiert ist, um kovalente Bindungen an ein Molekül von biologischem Interesse zu ermöglichen.

16. Verfahren nach einem der Ansprüche 1 bis 8, bei dem eine der Verbindungen in wenigstens einer der chemilumineszenten Reaktionen auf einer der folgenden Verbindungen basiert:
i) Phthalhydrazide und verwandte Verbindungen,
ii) Dioxetane, Dioxetanone und verwandte Verbindungen; und
iii) Bis-oxalsäureester, verwandte Verbindungen und, falls erforderlich, assoziierte Akzeptorpartner.

## Revendications (Revendications pour l'(les) Etat(s) contractant(s) suivant(s): AT, BE, CH, DE, DK, FR, GB, IT, LI, LU, NL, SE)

1. Procédé pour l'essai, la détection, la quantification, la localisation ou l'analyse d'un échantillon contenant au moins deux substances d'intérêt comprenant les étapes consistant à :
(i) faire réagir ledit échantillon avec un mélange comprenant au moins un premier réactif et un second réactif qui forment au moins un premier complexe et un second complexe, ledit premier complexe comprenant l'une desdites substances ou une substance associée respective et ledit premier réactif, et ledit second complexe comprenant une autre substance précitée et ledit second réactif, au moins ledit premier réactif et ledit second réactif comprenant chacun :
(a) un partenaire de liaison pour se lier ou autrement s'attacher à une ou plusieurs desdites substances ; et
(b) une molécule chimiluminescente différente présentant des caractéristiques d'émission distinguables, ladite molécule chimiluminescente étant chimiquement ou physiquement couplée audit partenaire de liaison ;
(ii) traiter ensuite ledit échantillon comprenant lesdits premier et second complexes pour amener une première et une seconde réaction chimiluminescente à se produire, lesdites réactions chimiluminescentes étant simultanément déclenchées ; et
(iii) observer, détecter, mesurer et/ou enregistrer les émissions de chacune desdites réactions chimiluminescentes.

2. Procédé selon la revendication 1, dans lequel les caractéristiques d'émission de chacune desdites réactions chimiluminescentes sont distinguables en termes de la variation d'intensité d'émission lumineuse ou de radiation avec la durée des émissions.

3. Procédé selon la revendication 2, dans lequel l'intensité de la radiation émise par ledit échantillon est observée, détectée, mesurée et/ou enregistrée sur deux intervalles de temps différents, lesdits intervalles de temps pouvant se chevaucher.

4. Procédé selon la revendication 1, dans lequel chacune desdites réactions chimiluminescentes émet une radiation dans un domaine spectral différent respectif.

5. Procédé selon la revendication 1, mettant en jeu au moins trois réactions chimiluminescentes, dans lequel au moins deux desdites réactions chimiluminescentes sont distinguables en termes des caractéristiques d'émission spectrale et dans lequel au moins deux desdites réactions chimiluminescentes sont distinguables en termes de la variation d'intensité de lumière ou de radiation au cours du temps.

6. Procédé selon l'une des revendications 4 ou 5, dans lequel les émissions sont filtrées par des moyens de filtrage respectifs sensibles à la radiation dans lesdits différents domaines spectraux, et les intensités filtrées des émissions sont observées, détectées, mesurées et/ou enregistrées.

7. Procédé selon la revendication 1 ou l'une quelconque des revendications dépendantes de celle-ci, dans lequel au moins l'un desdits premier et second complexes est composé d'un réactif lié à une substance par l'une parmi une réaction de liaison d'immuno-essai, une réaction de liaison à une protéine, une hybridation d'acide nucléique et une réaction de liaison à un récepteur.

8. Procédé selon l'une quelconque des revendications précédentes, dans lequel chacune desdites réactions chimiluminescentes comprend un composant ou réactif respectif qui est une variante structurale du ou de chaque composant ou réactif associé à la ou chaque autre réaction chimiluminescente.

9. Procédé selon l'une quelconque des revendications précédentes, dans lequel l'un des composants dans au moins l'une desdites réactions chimiluminescentes est à base de composés d'acridinium ou des variantes structurales de ceux-ci.

10. Procédé selon la revendication 9, dans lequel l'un des composants dans au moins l'une desdites réactions chimiluminescentes est un sel d'acridinium alkylé sur l'azote de noyau.

11. Procédé selon la revendication 10, dans lequel l'un desdits composants dans au moins l'une desdites réactions chimiluminescentes est un ester phénylique d'acridinium, l'ester étant formé en position 9 du noyau acridine.

12. Procédé selon la revendication 11, dans lequel l'une desdites réactions chimiluminescentes comprend un ester phénylique d'acridinium dans lequel la fraction phényle est substituée par des groupes extracteurs d'électrons pour donner une réaction chimiluminescente dans laquelle l'émission de lumière a lieu sur une période relativement courte, et dans lequel une autre desdites réactions chimiluminescentes comprend un ester phénylique d'acridinium dans lequel la fraction phényle est substituée par des groupes donneurs d'électrons pour donner une réaction chimiluminescente dans laquelle l'émission de lumière a lieu sur une période relativement longue.

13. Procédé selon la revendication 10, dans lequel l'une desdites réactions chimiluminescentes comprend un sel d'acridinium qui, lors du déclenchement de ladite réaction, émet une radiation à une longueur d'onde relativement courte, et dans lequel une autre desdites réactions chimiluminescentes comprend un sel d'acridinium dans lequel la conjugaison électronique du noyau acridine est accrue, ce par quoi, lors du déclenchement de ladite autre réaction, ledit sel d'acridinium émet à une longueur d'onde relativement longue.

14. Procédé selon la revendication 13, dans lequel ladite réaction chimiluminescente émet une radiation d'une longueur d'onde se situant dans la plage de 400 à 500 nm, et ladite autre réaction chimiluminescente émet une radiation d'une longueur d'onde se situant dans la plage de 500 à 700 nm.

15. Procédé selon la revendication 9 ou l'une quelconque des revendications dépendantes de celle-ci, dans lequel le composé d'acridinium est modifié ou transformé en un nouveau dérivé pour permettre un couplage covalent à une molécule d'intérêt biologique.

16. Procédé selon l'une quelconque des revendications 1 à 8, dans lequel l'un des composants dans au moins l'une desdites réactions chimiluminescentes est à base de l'un des composés suivants :
(i) phtalhydrazides et composés apparentés ;
(ii) dioxétanes, dioxétanones et composés apparentés ; et
(iii) esters bis-oxalates, composés apparentés et partenaires accepteurs associés si nécessaire.

17. Réactif luminescent qui comprend un mélange d'au moins deux réactifs, lesdits réactifs comprenant chacun :
(a) un partenaire de liaison pour se lier ou autrement s'attacher aux substances respectives d'intérêt ; et
(b) une molécule chimiluminescente différente présentant des caractéristiques d'émission distinguables, ladite molécule chimiluminescente étant chimiquement ou physiquement couplée au partenaire de liaison.

18. Réactif luminescent selon la revendication 17, dans lequel lesdites émissions chimiluminescentes sont chacune distinguables les unes des autres en termes de la variation d'intensité de lumière avec la durée des émissions.

19. Réactif luminescent selon la revendication 17, dans lequel chacune desdites émissions chimiluminescentes émet une radiation dans un domaine spectral différent.

20. Réactif luminescent selon la revendication 17 ou la revendication 18, dans lequel au moins l'un desdits réactifs est capable de se lier ou autrement s'attacher dans une réaction choisie dans le groupe constitué par une réaction de liaison d'immuno-essai, une réaction de liaison à une protéine, une réaction d'hybridation d'acide nucléique et une réaction de liaison à un récepteur.

21. Réactif luminescent selon l'une quelconque des revendications 17, 18, 19 et 20, dans lequel lesdites molécules chimiluminescentes sont des variantes structurales de la ou de chaque autre molécule chimiluminescente.

22. Réactif luminescent selon l'une quelconque des revendications 17, 18, 19 et 20, dans lequel l'une des molécules chimiluminescentes capables de prendre part dans au moins une réaction chimiluminescente respective est à base de composés d'acridinium ou de variantes structurales de ceux-ci.

23. Réactif luminescent selon la revendication 22, dans lequel l'une des molécules chimiluminescentes dans au moins l'une desdites réactions chimiluminescentes est un sel d'acridinium alkylé sur l'azote de noyau.

24. Réactif luminescent selon la revendication 22, dans lequel l'une desdites molécules chimiluminescentes dans au moins l'une desdites réactions chimiluminescentes est un ester phénylique d'acridinium, l'ester étant formé en position 9 du noyau acridine.

25. Réactif luminescent selon la revendication 24, dans lequel l'une desdites réactions chimiluminescentes comprend un ester phénylique d'acridinium dans lequel la fraction phényle est substituée par des groupes extracteurs d'électrons pour donner une réaction chimiluminescente dans laquelle l'émission de lumière a lieu sur une période relativement courte, et dans lequel une autre desdites réactions chimiluminescentes comprend un ester phénylique d'acridinium dans lequel la fraction phényle est substituée par des groupes donneurs d'électrons pour donner une réaction chimiluminescente dans laquelle l'émission de lumière a lieu sur une période relativement longue.

26. Réactif luminescent selon la revendication 22, dans lequel l'une desdites réactions chimiluminescentes comprend un sel d'acridinium qui, lors du déclenchement de ladite réaction, émet une radiation à une longueur d'onde relativement courte, et dans lequel une autre desdites réactions chimiluminescentes comprend un sel d'acridinium dans lequel la conjugaison électronique du noyau acridine est accrue, ce par quoi, lors du déclenchement de ladite autre réaction, ledit sel d'acridinium émet une radiation à une longueur d'onde relativement longue.

27. Réactif luminescent selon la revendication 26, dans lequel ladite réaction chimiluminescente émet une radiation d'une longueur d'onde se situant dans la plage de 400 à 500 nm, et ladite autre réaction chimiluminescente émet une radiation se situant dans la plage de 500 à 700 nm.

28. Réactif luminescent selon la revendication 22 ou selon l'une quelconque des revendications dépendantes de celle-ci, dans lequel le composé d'acridinium est modifié ou transformé en un nouveau dérivé pour permettre un couplage covalent à une molécule d'intérêt biologique.

29. Réactif luminescent selon l'une quelconque des revendications 17, 18, 19 et 20, dans lequel lesdits molécules chimiluminescentes sont capables de prendre part aux réactions chimiluminescentes respectives, au moins l'une desdites réactions chimiluminescentes étant basée sur l'un des composés suivants :
(i) phtalhydrazides et composés apparentés ;
(ii) dioxétanes, dioxétanones et composés apparentés ; et
(iii) esters bis-oxalates, composés apparentés et partenaires accepteurs associés si nécessaire.

## Revendications (Revendications pour l'(les) Etat(s) contractant(s) suivant(s): ES)

1. Procédé pour l'essai, la détection, la quantification, la localisation ou l'analyse d'un échantillon contenant au moins deux substances d'intérêt comprenant les étapes consistant à :
(i) faire réagir ledit échantillon avec un mélange comprenant au moins un premier réactif et un second réactif qui forment au moins un premier complexe et un second complexe, ledit premier complexe comprenant l'une desdites substances ou une substance associée respective et ledit premier réactif, et ledit second complexe comprenant une autre substance précitée et ledit second réactif, au moins ledit premier réactif et ledit second réactif comprenant chacun :
(a) un partenaire de liaison pour se lier ou autrement s'attacher à une ou plusieurs desdites substances ; et
(b) une molécule chimiluminescente différente présentant des caractéristiques d'émission distinguables, ladite molécule chimiluminescente étant chimiquement ou physiquement couplée audit partenaire de liaison ;
(ii) traiter ensuite ledit échantillon comprenant lesdits premier et second complexes pour amener une première et une seconde réaction chimiluminescente à se produire, lesdites réactions chimiluminescentes étant simultanément déclenchées ; et
(iii) observer, détecter, mesurer et/ou enregistrer les émissions de chacune desdites réactions chimiluminescentes.

2. Procédé selon la revendication 1, dans lequel les caractéristiques d'émission de chacune desdites réactions chimiluminescentes sont distinguables en termes de la variation d'intensité d'émission lumineuse ou de radiation avec la durée des émissions.

3. Procédé selon la revendication 2, dans lequel l'intensité de la radiation émise par ledit échantillon est observée, détectée, mesurée et/ou enregistrée sur deux intervalles de temps différents, lesdits intervalles de temps pouvant se chevaucher.

4. Procédé selon la revendication 1, dans lequel chacune desdites réactions chimiluminescentes émet une radiation dans un domaine spectral différent respectif.

5. Procédé selon la revendication 1, mettant en jeu au moins trois réactions chimiluminescentes, dans lequel au moins deux desdites réactions chimiluminescentes sont distinguables en termes des caractéristiques d'émission spectrale et dans lequel au moins deux desdites réactions chimiluminescentes sont distinguables en termes de la variation d'intensité de lumière ou de radiation au cours du temps.

6. Procédé selon l'une des revendications 4 ou 5, dans lequel les émissions sont filtrées par des moyens de filtrage respectifs sensibles à la radiation dans lesdits différents domaines spectraux, et les intensités filtrées des émissions sont observées, détectées, mesurées et/ou enregistrées.

7. Procédé selon la revendication 1 ou l'une quelconque des revendications dépendantes de celle-ci, dans lequel au moins l'un desdits premier et second complexes est composé d'un réactif lié à une substance par l'une parmi une réaction de liaison d'immuno-essai, une réaction de liaison à une protéine, une hybridation d'acide nucléique et une réaction de liaison à un récepteur.

8. Procédé selon l'une quelconque des revendications précédentes, dans lequel chacune desdites réactions chimiluminescentes comprend un composant ou réactif respectif qui est une variante structurale du ou de chaque composant ou réactif associé à la ou chaque autre réaction chimiluminescente.

9. Procédé selon l'une quelconque des revendications précédentes, dans lequel l'un des composants dans au moins l'une desdites réactions chimiluminescentes est à base de composés d'acridinium ou des variantes structurales de ceux-ci.

10. Procédé selon la revendication 9, dans lequel l'un des composants dans au moins l'une desdites réactions chimiluminescentes est un sel d'acridinium alkylé sur l'azote de noyau.

11. Procédé selon la revendication 10, dans lequel l'un desdits composants dans au moins l'une desdites réactions chimiluminescentes est un ester phénylique d'acridinium, l'ester étant formé en position 9 du noyau acridine.

12. Procédé selon la revendication 11, dans lequel l'une desdites réactions chimiluminescentes comprend un ester phénylique d'acridinium dans lequel la fraction phényle est substituée par des groupes extracteurs d'électrons pour donner une réaction chimiluminescente dans laquelle l'émission de lumière a lieu sur une période relativement courte, et dans lequel une autre desdites réactions chimiluminescentes comprend un ester phénylique d'acridinium dans lequel la fraction phényle est substituée par des groupes donneurs d'électrons pour donner une réaction chimiluminescente dans laquelle l'émission de lumière a lieu sur une période relativement longue.

13. Procédé selon la revendication 10, dans lequel l'une desdites réactions chimiluminescentes comprend un sel d'acridinium qui, lors du déclenchement de ladite réaction, émet une radiation à une longueur d'onde relativement courte, et dans lequel une autre desdites réactions chimiluminescentes comprend un sel d'acridinium dans lequel la conjugaison électronique du noyau acridine est accrue, ce par quoi, lors du déclenchement de ladite autre réaction, ledit sel d'acridinium émet à une longueur d'onde relativement longue.

14. Procédé selon la revendication 13, dans lequel ladite réaction chimiluminescente émet une radiation d'une longueur d'onde se situant dans la plage de 400 à 500 nm, et ladite autre réaction chimiluminescente émet une radiation d'une longueur d'onde se situant dans la plage de 500 à 700 nm.

15. Procédé selon la revendication 9 ou l'une quelconque des revendications dépendantes de celle-ci, dans lequel le composé d'acridinium est modifié ou transformé en un nouveau dérivé pour permettre un couplage covalent à une molécule d'intérêt biologique.

16. Procédé selon l'une quelconque des revendications 1 à 8, dans lequel l'un des composants dans au moins l'une desdites réactions chimiluminescentes est à base de l'un des composés suivants :
(i) phtalhydrazides et composés apparentés;
(ii) dioxétanes, dioxétanones et composés apparentés ; et
(iii) esters bis-oxalates, composés apparentés et partenaires accepteurs associés si nécessaire.
